# EUROPÄISCHE PATENTSCHRIFT

(11) **EP 1 242 346 B1**
(45) Veröffentlichungstag und Bekanntmachung des Hinweises auf die Patenterteilung: **21.06.2006**
(21) Anmeldenummer: 00985231.0
(22) Anmeldetag: 21.12.2000
(51) Int. Cl.: C07C 29/80, C07C 29/141, C07C 31/22

(54) **VERFAHREN ZUR REINIGUNG VON DURCH HYDRIERUNG HERGESTELLTEM TRIMETHYLOLPROPAN DURCH KONTINUIERLICHE DESTILLATION**
METHOD FOR PURIFYING TRIMETHYLOLPROPANE, WHICH IS PRODUCED BY HYDROGENATION, BY MEANS OF CONTINUOUS DISTILLATION
PROCEDE DE PURIFICATION, PAR DISTILLATION CONTINUE, DE TRIMETHYLPROPANE OBTENU PAR HYDROGENATION

(30) Priorität: 28.12.1999 DE 19963435
(43) Veröffentlichungstag der Anmeldung: 25.09.2002
(73) Patentinhaber: BASF Aktiengesellschaft, 67056 Ludwigshafen (DE)
(72) Erfinder: DERNBACH, Matthias, 69221 Dossenheim (DE); KRATZ, Detlef, 69121 Heidelberg (DE); STAMMER, Achim, Mobile, AL 36695 (US); RUST, Harald, 67435 Neustadt (DE); SCHULZ, Gerhard, 67098 Bad Dürkheim, (DE)
(74) Vertreter: Isenbruck, Günter
(86) Internationale Anmeldenummer: PCT/EP2000/013105
(87) Internationale Veröffentlichungsnummer: WO 2001/047847

(56) Entgegenhaltungen:
- DD-A- 45 078
- DE-A- 19 848 568
- DE-A- 19 848 569

## Beschreibung

Die vorliegende Anmeldung bezieht sich auf das Gebiet der industriellen Chemie. Genauer gesagt, betrifft sie ein Verfahren, bei dem Trimethylolpropan, das durch Hydrierung von Dimethylolbutanal erhalten wurde, destillativ gereinigt wird.

Trimethylolpropan, nachfolgend TMP abgekürzt, ist ein dreiwertiger Alkohol, der ein weites Anwendungsfeld in der Herstellung von Lacken, Polyurethanen und Polyestern wie beispielsweise Alkydharzen, gefunden hat. Trimethylolpropan wird hergestellt durch Kondensationsreaktion von n-Butyraldehyd mit Formaldehyd. Je nach Verfahrensführung unterscheidet man dabei zwischen verschiedenen Varianten.

Zum einen existiert das sogenannte Cannizzaro-Verfahren, bei dem der Butyraldehyd mit dem Formaldehyd unter Zugabe von stöchiometrischen Mengen einer Base umgesetzt wird. Es entsteht dabei in der ersten Stufe 2,2-Dimethylolbutanal, das anschliessend in einer sogenannten gekreuzten Cannizzaro-Reaktion mit überschüssigem Formaldehyd zu Ameisensäure und Trimethylolpropan reagiert. Je nach Art der Base spricht man dabei vom anorganischen bzw. organischen Cannizzaro-Verfahren. Bei der anorganischen Verfahrensweise wird eine anorganische Base, meistens NaOH oder Ca(OH)₂ benutzt. Der Nachteil bei dieser Verfahrensweise ist, daß eine Menge an unerwünschten Nebenprodukten entsteht, die schwer abzutrennen sind und bei der weiteren Verwendung des Trimethylolpropans stören. Weiterhin bildet sich ein Moläquivalent an Ameisensäuresalz, das verworfen werden muß und somit den Verbrauch an Formaldehyd erhöht sowie zur Umweltbelastung beiträgt.

Beim organischen Cannizzaro-Verfahren wird anstelle der anorganischen Base ein tertiäres Amin, generell ein Trialkylamin, eingesetzt. Die Reaktion verläuft wie oben dargelegt, wobei ein Äquivalent Ammoniumformiat des entsprechenden Amins gebildet wird. Dieses kann durch entsprechende Maßnahmen weiter aufgearbeitet werden, wodurch zumindest das Amin wiedergewonnen und in die Reaktion zurückgeführt werden kann. Das erhaltene Roh-TMP kann auf verschiedene Weise zu reinem TMP aufgearbeitet werden.

Eine Weiterentwicklung ist das Hydrierverfahren, in dem Butyraldehyd und Formaldehyd nicht in Gegenwart von mindestens stöchiometrischen Mengen, sondern von katalytischen Mengen eines tertiären Amins, im Allgemeinen ca. 5 bis 10 Mol-%, miteinander zur Reaktion gebracht werden. Dabei bleibt die Reaktion auf der Stufe von 2,2-Dimethylolbutanal stehen, das anschliessend durch Hydrierung in Trimethylolpropan überführt wird. Hierbei fallen keine stöchiometrischen Mengen eines Formiats an und die erhaltene Lösung ist leichter zu reinigen, da weniger störende Nebenprodukte entstehen. Es müssen jedoch in vielen Fällen reaktionstechnische Maßnahmen ergriffen werden, um eine vollständige Umsetzung der Edukte zum Dimethylolbutanal zu erreichen. Die Beschreibung eines effektiven Verfahrens findet sich in der WO 98/28253 der Anmelderin.

Der Stand der Technik ist reich an Druckschriften, die verschiedene Techniken zur Aufarbeitung von Trimethylolpropan beschreiben. Beim Studium dieser Druckschriften werden die Unterschiede in der Aufarbeitung deutlich, die die verschiedenen Herstellungsweisen für Trimethylolpropan erfordern. Die folgenden Druckschriften beziehen sich auf die Reinigung von Trimethylolpropan, das nach dem anorganischen Cannizzaro-Verfahren erhalten wurde.

Die DD-P- 45 078 offenbart ein Verfahren, in dem das erhaltene Roh-TMP mit einem sekundären cycloaliphatischen Alkohol, beispielsweise Cyclohexanol, versetzt wird, man dann Wasser zusammen mit diesem Alkohol azeotrop abdestilliert, und die ausgefallenen Formiate durch Filtration abtrennt. Nach Abdestillieren des überschüssigen Alkohols wird dann das erhaltene Rohprodukt zur Reinigung destilliert.

In der DD-P- 287 251 ist ein Verfahren zum Befreien von TMP von gebildeten Hochsiedern beschrieben. Die Hochsieder sammeln sich bei der Vakuumdestillation von Roh-TMP in den Fraktionen an, die schwerer flüchtig als TMP sind. Durch Zusatz von 0,02 bis 0,05 kg Säure/kg Destillat lassen sich viele der Hochsieder, die aus Reaktionsfolgeprodukten von TMP, insbesondere Formalen, bestehen, wieder in TMP überführen. Eine Ausbeutesteigerung wird erreicht.

Auch die GB 1 290 036 beschreibt ein Verfahren zum Zersetzen von Formalen des TMP in nach dem anorganischen Cannizzaro-Verfahren erhaltenen Rohchargen. Durch Zugabe von Kationenaustauscherharzen und Erhitzen werden in den Rohgemischen vorhandene Formale, die einen ähnlichen Siedepunkt wie TMP haben, in Produkte anderen Siedepunkts überführt, die leicht destillativ abzutrennen sind. Reines TMP kann erhalten werden.

Die US 3,097,245 beschreibt ein Verfahren zur Herstellung von Trimethylolpropan mit einer APHA-Farbzahl zwischen 50 und 200. Diese Farbzahl wird dabei durch das Einhalten bestimmter Reaktionsbedingungen hinsichtlich Temperatur, Reaktionszeit, pH-Wert und Konzentration der Ausgangsverbindungen erreicht. Weiterhin schließt sich an die Reaktion die Behandlung der erhaltenen Lösung mit Ionenaustauscherharzen an.

Die US 5,603,835 offenbart ein Verfahren zur Herstellung von TMP mit APHA-Farbzahlen von < 100. Diese werden erreicht durch extraktive Nachbehandlung der erhaltenen rohen TMP-Lösungen mit einem Ether oder einem Ester. Die eingesetzten TMP-Lösungen stammen im Allgemeinen aus dem anorganischen Cannizzaro-Verfahren.

Demgegenüber wird Roh-TMP, das aus dem organischen Cannizzaro-Verfahren stammt, anders aufgearbeitet.

In der DE-P-142 090 findet sich die Beschreibung der Aufarbeitung eines solchen Roh-TMP-Gemisches. Dieses Roh-Gemisch wird dabei destillativ aufgearbeitet, anschliessend hydriert und erneut destilliert. Ein solches Verfahren ist aufwendig, erfordert ein hohes Vakuum und erbringt geringe Ausbeuten.

Bei der Herstellung von TMP nach dem organischen Cannizzaro-Verfahren existiert insbesondere eine störende Nebenreaktion, die die Ausbeute an TMP deutlich herabsetzen kann. Das in der Reaktion gebildete Trialkylammoniumformiat reagiert unter bestimmten Bedingungen, wie beispielsweise dem Entwässern der Lösung oder Erhitzen, zu Trialkylamin und Trimethylolpropanformiaten. Diese schmälern die Ausbeute an Trimethylolpropan und sollten daher wieder möglichst vollständig gespalten werden, ohne daß dabei unerwünschte Nebenreaktionen auftreten.

Ein dazu geeignetes Verfahren findet sich in der WO 97/17313. Dabei wird im ersten Schritt in an sich bekannter Weise Trimethylolpropan durch Reaktion von Formaldehyd mit Butyraldehyd in Gegenwart von stöchiometrischen Mengen eines tertiären Amins hergestellt. Das TMP-Rohgemisch wird im zweiten Schritt von überschüssigem Wasser, tertiärem Amin und Formaldehyd befreit. Im dritten Schritt wird das danach verbliebene Gemisch erhitzt, wobei die Spaltung des Trialkylammoniumformiats in Trialkylamin und Ameisensäure (die beide abgetrennt werden) und Bildung von TMP-Formiaten eintritt. Im vierten Schritt wird das abgetrennte Amin zurückgeführt, und zwar in den ersten oder den nachfolgenden fünften Schritt. In diesem fünften Schritt wird das erhaltene TMP-Formiat mit einem niederen Alkohol, unter Katalyse eben des abgetrennten Amins, umgesetzt, wobei das TMP unter Bildung von Methylformiat freigesetzt wird.

Ein ähnliches Verfahren ist in der DE-A-198 48 568 offenbart. Das nach der üblichen Reaktion in Gegenwart von stöchiometrischen Mengen eines Trialkylamins erhaltene TMP-Rohgemisch wird erhitzt, von Trialkylamin befreit und, bevor es destillativ aufgearbeitet wird, mit entweder Wasser, Ammoniak, einem primären oder einem sekundären Amin versetzt. Das beim Erhitzen gebildete Trimethylolpropanformiat wird in TMP und Ameisensäure bzw. ein Formamid überführt. Die Ausbeute an TMP wird gesteigert.

In der DE 19848569 wird das nach der bekannten Methode mittels stöchiometrischer Zugabe eines Trialkylamins erhaltene TMP-Rohgemisch nach Entfernung des überschüssigen Wassers und Methanols unter Druck erhitzt. Unter diesen Bedingungen zerfällt das gebildete Trialkylammoniumformiat in Trialkylamin, Wasserstoff und Kohlendioxid und/oder Wasser und Kohlenmonoxid. Das Reaktionsgemisch enthält kein Formiat des Trimethylolpropan.

Die vorstehend genannten Verfahren eignen sich jedoch nur bedingt zur effektiven Aufarbeitung eines nach dem sogenannten Hydrierverfahren erhaltenen TMP-Gemischs, in dem lediglich katalytische Mengen an Trialkylamin verwendet werden und das somit auch nur geringe Mengen an Trialkylammoniumformiat enthält. Es ist daher die Aufgabe der vorliegenden Erfindung, ein solches Verfahren bereitzustellen. Dieses Verfahren soll es weiterhin ermöglichen, TMP mit hoher Reinheit, vorzugsweise > 99 %, mit einer geringen Farbzahl von 10 bis 100 APHA und in hoher Ausbeute herzustellen.

Diese Aufgabe wird gelöst durch ein Verfahren zur Reindestillation von aus der Hydrierung von 2,2-Dimethylolbutanal stammendem Trimethylolpropan, das die folgenden Schritte umfasst:
a) Umsetzung von n-Butyraldehyd mit Formaldehyd in Gegenwart von katalytischen Mengen eines tertiären Amins und Hydrieren der so erhaltenen Mischung zu einem Trimethylolpropan enthaltenden Gemisch,
b) destillative Abtrennung von Wasser, Methanol, Trialkylamin und/oder Trialkylammoniumformiat bei Drücken von > 200 mbar, vorzugsweise > 400 mbar, Sumpftemperaturen von > 140°C, vorzugsweise 160-180°C, sowie langen Verweilzeiten so durchgeführt wird, dass zumindest der überwiegende Teil des TMP mit in Schritt (a) gebildetem Trialkylammoniumformiat zu im Sumpf verbleibenden TMP-Formiaten und mit Wasser und Methanol abdestillierendem Trialkylamin reagiert.
c) Erhitzen des in b) erhaltenen Rückstands im Vakuum auf eine Temperatur, bei der Trimethylolpropan flüchtig ist und höher als Trimethylolpropan siedende Verbindungen zumindest teilweise gespalten werden, unter destillativem Abtrennen von Trimethylolpropan sowie leichter als Trimethylolpropan flüchtigen Verbindungen;
d) Destillation des in c) erhaltenen Destillats unter Abtrennen der leichter flüchtigen Verbindungen und Gewinnen von Rein-TMP;
e) fakultative Destillation des in d) erhaltenen Trimethylolpropans zum Gewinnen von TMP mit niedriger APHA-Farbzahl.

Im Rahmen des erfindungsgemäßen Verfahrens zur Reindestillation von Trimethylolpropan werden TMP-Rohlösungen gereinigt, die nach dem sogenannten Hydrierverfahren hergestellt wurden. Das TMP wurde also erhalten durch Kondensation von n-Butyraldehyd mit Formaldehyd in Gegenwart von katalytischen Mengen eines tertiären Amins und anschliessender katalytischer Hydrierung des entstandenen Dimethylolbutanal-Gemischs. Das Roh-TMP enthält also keine Alkali- oder Erdalkaliformiate oder sonstige Verunreinigungen, die bei dem anorganischen Cannizzaro-Verfahren entstehen. Ebenfalls enthält das Roh-TMP nur geringe Mengen, ca. 5 bis 10 Mol-%, an Trialkylamrnoniumformiaten bzw. freiem Trialkylamin, anders als beim organischen Cannizzaro-Verfahren.

Das der Hydrierung entstammende und dem erfindungsgemäßen Reinigungsverfahren zu unterwerfende Roh-TMP enthält neben Trimethylolpropan und Wasser noch Methanol, Trialkylamin, Trialkylammoniumformiat, länger-kettige lineare und verzweigte Alkohole und Diole, beispielsweise Methylbutanol oder Ethylpropandiol, Additionsprodukte von Formaldehyd und Methanol an Trimethylolpropan, Acetale wie Dimethylolbutyraldehyd-TMP-Acetal sowie das sogenannte Di-TMP.

Gute Ergebnisse wurden erzielt mit Hydrierausträgen, die 10 bis 40 Gew.-% Trimethylolpropan, 0,5 bis 5 Gew.-% Methanol, 1 bis 6 Gew.-% Methylbutanol, 1 bis 10 Gew.-% Trialkylammoniumformiat, 0 bis 5 Gew.-% 2-Ethylpropandiol, 2 bis 10 Gew.-% Hochsieder wie Di-TMP oder auch andere Additionsprodukte und 5 bis 80 Gew.-% Wasser aufweisen. Hydrierausträge einer derartigen Zusammensetzung können beispielswiese erhalten werden nach dem in der WO 98/28253 beschriebenen Verfahren.

In dem der Hydrierung nachfolgenden Verfahrensschritt b) wird dann der Hydrieraustrag einer Destillation unterworfen, bei der Wasser und andere leicht flüchtige Verbindungen wie Methanol, Trialkylamin und gegebenenfalls Trialkylammoniumformiat abgetrennt werden. Diese Destillation wird mit den einem Fachmann bekannten Apparaturen durchgeführt, beispielsweise Verdampfern und/oder Destillationskolonnen.

Das Abdestillieren von Wasser und den anderen Leichtsiedern geschieht dagegen bei Drücken von > 200 mbar, vorzugsweise > 400 mbar und Sumpftemperaturen von > 140°C, vorzugsweise 160 bis 185°C. Dabei findet eine Umsetzung des Trialkylammoniumformiats mit Trimethylolpropan zu Trialkylamin und Formiaten des Trimethylolpropans statt. Lange Verweilzeiten begünstigen ebenfalls die Bildung dieser Formiate. Das gebildete Trialkylamin destilliert mit den anderen Leichtsiedern ab und kann in der Aldolisierungsreaktion wiederverwendet werden. Im nach der Destillation erhaltenen Sumpf befinden sich dann ca. 2 bis 10 Gew.-% Trimethylolpropanformiat.

An die Stufe (b) kann sich ein Verfahrensschritt (bb) anschließen, in dem diese Formiate gespalten werden und TMP zurückgewonnen wird.

Dies kann-beispielsweise in an sich bekannter Weise dadurch geschehen, daß eine Umesterung mit einem niederen Alkohol, beispielsweise Methanol, unter Bildung von Formiaten dieses Alkohols und TMP durchgeführt wird. Diese Umesterung kann durchgeführt werden wie beispielsweise in der EP-A- 289 921 beschrieben, in Gegenwart katalytischer Mengen an Alkali- oder Erdalkalialkoholaten. Möglich ist auch eine Reaktionsführung, wie sie in der WO 97/ 17313 offenbart wird. Hier wird ein tertiäres Amin als Katalysator der Umsetzung eingesetzt. Ebenfalls möglich ist die säurekatalysierte Umsetzung.

Ebenfalls kann die Freisetzung von Trimethylolpropan aus seinen Formiaten durch Umsetzen mit einem wasserfreien sekundären Amin geschehen, wie dies in der deutschen Anmeldung mit dem Titel "Verfahren zur Umwandlung von bei der Trimethylolalkan-Herstellung anfallenden Trimethylolalkanformiat" (Anmelderin: BASF AG) beschrieben wurde.

Im Verfahrensschritt (c) wird anschließend der aus den Schritten (b) bzw. (bb) hervorgehende Rückstand auf eine Temperatur erhitzt, bei der die sogenannten Hochsieder, das heißt schwerer als TMP flüchtige Verbindungen, abgetrennt werden. Dieses Erhitzen geschieht im Vakuum, bei Drücken von 5 bis 50 mbar, vorzugsweise 10 bis 30 mbar. Die Sumpftemperaturen betragen dabei 210 bis 250 °C, vorzugsweise 220 bis 235°C. Bei diesen Bedingungen destilliert das TMP zusammen mit anderen, leichter als TMP flüchtigen Verbindungen, den sogenannten Leichtsiedern, ab. Wichtig ist im Zusammenhang mit der vorliegenden Erfindung, daß durch die hohen Temperaturen im Sumpf die Spaltung eines Teils der Hochsieder, die Derivate von TMP sind, erreicht wird. Dies sind beispielsweise Dimethylolbutyraldehyd-TMP-Acetal und höhere Acetale. Durch geeignete, an sich bekannte Maßnahmen, beispielsweise die Zugabe von Säure, kann die Zersetzung der Hochsieder beschleunigt werden. Dadurch läßt sich die Ausbeute an TMP nochmals steigern. Besonders vorteilhaft ist eine Säurezugabe gemäß dem in der deutschen Anmeldung mit dem Titel "Verfahren zum Zersetzen von bei der Synthese mehrwertiger Alkohole gebildeter hochsiedender Nebenprodukte" Aktenzeichen 199 63 437.8 (Anmelderin: BASF AG) beschriebenen Verfahren. Man erhält einen Sumpf mit einem Gehalt von 1 bis 50 Gew.-% TMP. Die Leichtsieder und TMP werden im Verfahrensschritt (c) von den im Sumpf verbleibenden Verunreinigungen gemeinsam abdestilliert und aufgefangen. Die Destillation wird dabei im Allgemeinen mittels einer Kolonne durchgeführt werden, wobei das Rücklaufverhältnis 0 bis 3, vorzugsweise 0 bis 1 beträgt. In der Kolonne werden die üblichen, einem Fachmann bekannten Einbauten verwendet. Vorzugsweise benutzt man geordnete Packungen.

Das aus dem Schritt (c) stammende Destillat, das TMP und die sogenannten Leichtsieder, wie beispielsweise 2-Ethylpropandiol oder auch TMP-Formiat enthält, wird dann in Schritt (d) einer Reindestillation unterworfen. Diese Destillation wird generell in einer Kolonne durchgeführt. Die Leichtsieder werden über Kopf abgetrennt, und das TMP wird als Seitenabzug der Kolonne entnommen. Vorzugsweise wird der Seitenabzug unterhalb des Zulaufs entnommen. Der Seitenabzug kann flüssig sein, es ist jedoch bevorzugt, wenn er gasförmig vorliegt.

Es kann dabei ein TMP mit einer Reinheit von > 99% und einer APHA-Farbzahl von 20 bis 200 erhalten werden. Die Destillation wird in den üblichen einem Fachmann bekannten Kolonnen vorgenommen, vorzugsweise Kolonnen, die mit Einbauten ausgestattet sind. Vorzugsweise werden geordnete Packungen verwendet. Die Destillation wird durchgeführt bei Drücken von 10 bis 40 mbar, vorzugsweise 20 bis 30 mbar und bei Sumpftemperaturen von 170 bis 210°C, vorzugsweise von 180 bis 200°C. Es hat sich als vorteilhaft erwiesen, aus dem Sumpf einen kleinen Strom auszuschleusen, um ein Ansammeln von hochsiedenden und/oder farbgebenden Komponenten zu verhindern.

In der Variante der vorliegenden Erfindung können die Schritte (b) und (d) miteinander kombiniert werden. Dabei wird so verfahren, daß das in Schritt (a) nach dem Hydrieren erhaltene Gemisch derart aufdestilliert wird, daß nicht nur die sehr leicht flüchtigen Verbindungen wie Wasser, Methanol und Triethylamin abdestilliert werden, sondern auch die anderen sogenannten Leichtsieder, die im Prinzip erst wie vorgehend beschrieben nach dem Abtrennen der Hochsieder vom TMP entfernt werden. Dabei handelt es sich beispielsweise um 2-Ethylpropandiol oder auch TMP-Formiate, wobei diese aber auch häufig teilweise schon mit den sehr leichtflüchtigen Verbindungen übergehen. Um ein solches vollständiges Abtrennen der leichter als TMP flüchtigen Verbindungen zu erreichen, wird die Abtrennung bei Drücken von 10 bis 40 mbar und Temperaturen von 170 bis 210°C durchgeführt. Die Abtrennung kann in Verdampfern durchgeführt werden. Bevorzugt wird die Verwendung von Destillationskolonnen, da so das Abdestillieren von TMP verhindert werden kann.

In einer weiteren Variante der Erfindung können die Stufen (c) und (d), also das gemeinsame destillative Abtrennen von TMP und Leichtsiedern von den Hochsiedern und das anschließende destillative Abtrennen der Leichtsieder von TMP, in einem einzigen Schritt durchgeführt werden. Dazu müssen beim Erhitzen des der Stufe (b) entnommenen Gemischs unter den oben erwähnten Bedingungen die flüchtigen Verbindungen über eine Kolonne mit geeigneter Trennleistung abdestilliert und mit dieser aufgetrennt werden. Vorteilhafterweise werden Kolonnen mit Seitenabzug verwendet, also solche, die auch bei der von Schritt (c) getrennten Durchführung des Schritts (d) verwendet werden.

Das aus der Stufe (d), also der Reindestillation zum Abtrennen der Leichtsieder, entnommene TMP kann einer zweiten Reindestillation (e) unterworfen werden. Diese zweite Destillation ist fakultativ und dient dem Verbessern der Farbzahl, sollte ein möglichst farbloses TMP gewünscht sein. Es kann gesagt werden, daß die Destillation (d) durchgeführt wird, um ein reines Produkt zu erhalten. Die Destillation (e) bewirkt praktisch keine Verbesserung der Reinheit mehr, sondern lediglich eine der Farbzahl.

Die Destillation wird generell in einer Kolonne durchgeführt. Leichter siedende, farbgebende Komponenten werden über Kopf abgetrennt, und das TMP wird als Seitenabzug der Kolonne entnommen. Vorzugsweise wird der Seitenabzug unterhalb des Zulaufs entnommen. Der Seitenabzug kann flüssig sein, es ist jedoch bevorzugt, wenn er gasförmig ist.

Es kann dabei ein TMP mit Farbzahlen von 10 bis 100 APHA erhalten werden. Die Destillation wird in den üblichen einem Fachmann bekannten Kolonnen vorgenommen, vorzugsweise in Kolonnen, die mit Einbauten ausgestattet sind. Als bevorzugte Einbauten werden geordnete Packungen verwendet. Die Destillation wird durchgeführt bei Drücken von 5 bis 40 mbar, vorzugsweise 20 bis 30 mbar, und Sumpftemperaturen von 170 bis 210°C, vorzugsweise 180 bis 200°C. Hierbei hat es sich als vorteilhaft erwiesen, aus dem Sumpf einen kleinen Strom auszuschleusen, da so ein Ansammeln von höhersiedenden und/oder farbgebenden Komponenten verhindert wird.

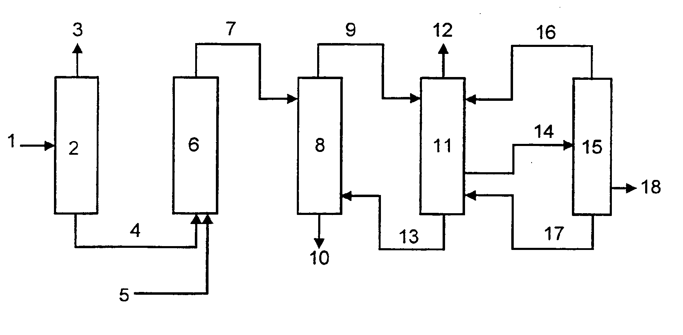

Das erfindungsgemäße Verfahren wird nun im Hinblick auf die in Figur 1 dargelegte Verfahrensskizze erläutert, die eine bestimmte Variante des Verfahrens veranschaulicht.

Die nach Kondensationsreaktion und nachfolgender Hydrierung erhaltene TMP-Rohlösung 1 wird in die Leichtsiederkolonne 2 eingetragen, in der ein Gemisch 3 aus Wasser und Leichtsiedern wie Methanol oder Trialkylamin abgetrennt wird. Dieses Trialkylamin kann, nach vorhergehendem Reinigen, erneut als Katalysator in die Kondensationsreaktion zwischen n-Butyraldehyd und Formaldehyd eingesetzt werden. Der der Leichtsiederkolonne entnommene Sumpf 4, der TMP, TMP-Formiate, Hochsieder und die nicht in der Kolonne 2 abgetrennten Leichtsieder enthält, wird in den Reaktor 6 verbracht, in dem Trimethylolpropanformiate gespalten werden. Dies geschieht durch Zugabe eines Dialkylamins oder von Alkohol wie etwa Methanol, gekennzeichnet durch die Nummer 5. In dem Reaktor 6 geschieht die Umsetzung der TMP-Formiate zu TMP und entweder Formiaten des eingesetzen Alkohols oder Formamiden des eingesetzten Amins.

Die an TMP-Formiaten verarmte Lösung 7 gelangt nun in die Hochsiederabtrennung 8, in der die Lösung 7 im Vakuum auf Temperaturen erhitzt wird, bei denen ein Gemisch 9 aus TMP und leichterflüchtigen Substanzen abdestilliert. Gleichzeitig werden durch die hohen Temperaturen hochsiedende Komponenten, die Derivate von TMP sind, zersetzt. Diese destillieren dann ebenfalls mit dem Gemisch 9 ab und erhöhen die Ausbeute an TMP. Es verbleibt ein Sumpf 10, der reich an Hochsiedern ist. Dieser Sumpf 10 wird verworfen oder kann zur Heizdampferzeugung verbrannt werden. Es ist auch möglich, diesen Sumpf 10 zur Reindestillation bestimmter Verbindungen weiter aufzureinigen.

Das TMP/Leichtsiedergemisch 9 wird dann in die erste Reindestillationsanlage 11 verbracht. Dabei werden über Kopf die leichter als TMP siedenden Verunreinigungen 12 abgetrennt. Diese können verworfen, zur Heizdampferzeugung verbrannt oder zur Gewinnung einzelner der enthaltenen Komponenten weiter aufgereinigt werden. Aus der Reindestillationsanlage 11 wird ein Strom 13 von farbgebenden und hochsiedenden Komponenten ausgeschleust, der in die Hochsiederabtrennung 8 zurückgeführt oder auch verworfen werden kann. Schließlich wird das der Anlage 11 entnommene Rein-TMP 14 einer (fakultativen) Destillation in einer Farbzahldestillationsvorrichtung 15 unterworfen. Dabei werden die leichtersiedenden, farbzahlgebenden Komponenten 16 über Kopf abdestilliert. Sie können verworfen werden oder in die Reindestillationsanlage 11 zurückgeführt werden.

Im Sumpf der Vorrichtung 15 sich langsam ansammelnde hochsiedende und farbgebende Komponenten 17 werden ausgeschleust und verworfen oder in die Reindestillationsanlage zurückgeführt. Es wird ein reines, farbzahlarmes TMP 18 gewonnen.

Die vorliegende Erfindung wird nun anhand der folgenden Beispiele erläutert. Dabei wurde in allen Beispielen ein Trimethylolpropan verwendet, das wie folgt dargestellt worden war:

Eine Apparatur bestehend aus zwei beheizbaren, durch Überlaufrohre miteinander verbundenen Rührkesseln mit einem Fassungsvermögen von insgesamt 72 1 wurde mit frischer, wäßriger Formaldehydlösung (4300 g/h in Form der 40%igen wäßrigen Lösung) und n-Butyraldehyd (1800 g/h) und mit frischem Trimethylamin als Katalysator (130 g/h) in Form der 45%igen wäßrigen Lösung kontinuierlich beschickt. Die Reaktoren wurden dabei auf 40°C temperiert.

Der Austrag wurde direkt in den oberen Teil eines Fallfilmverdampfers mit aufgesetzter Kolonne (11 bar Heizdampf) geleitet und dort bei normalem Druck destillativ in ein leichtsiedendes Kopfprodukt, im wesentlichen enthaltend n-Butyraldehyd, Ethylacrolein, Formaldehyd, Wasser und Trimethylamin, und ein hochsiedendes Sumpfprodukt aufgetrennt.

Das Kopfprodukt wurde kontinuierlich kondensiert und in die oben beschriebenen Reaktoren zurückgeführt.

Das hochsiedende Sumpfprodukt aus dem Verdampfer (ca. 33,5 kg/h) wurde kontinuierlich mit frischem Trimethylamin-Katalysator (50 g/h, in Form der 45%igen wäßrigen Lösung) versetzt und in einen beheizbaren, mit Füllkörpern versehenen Rohrreaktor mit einem Leervolumen von 12 1 geführt. Der Reaktor war dabei auf 40°C temperiert.

Der Austrag des Nachreaktors wurde kontinuierlich in den oberen Teil einer weiteren Destillationseinrichtung, der Formaldehydabtrennung, (11 bar Heizdampf) gegeben und dort destillativ in ein leichtsiedendes Kopfprodukt, im wesentlichen enthaltend Ethylacrolein, Formaldehyd, Wasser und Trimethylamin, und ein hochsiedendes Sumpfprodukt aufgetrennt. Das leichtsiedende Kopfprodukt (27 kg/h) wurde kontinuierlich kondensiert und in den ersten Rührkessel zurückgeleitet, wohingegen das hochsiedende Sumpfprodukt gesammelt wurde.

Das so erhaltene Sumpfprodukt enthielt neben Wasser im wesentlichen Dimethylolbutyraldehyd, Formaldehyd und Spuren Monomethylbutyraldehyd. Es wurde dann einer kontinuierlichen Hydrierung unterworfen. Dazu wurde die Reaktionslösung bei 90 bar und 115°C in einem Hauptreaktor in Kreislauf/Rieselfahrweise und einem nachgeschalteten Nachreaktor in Kreislauffahrweise hydriert. Der Katalysator wurde analog D der DE 198 09 418 hergestellt. Er enthielt 24 % CuO, 20 % Cu und 46 % TiO₂. Die verwendete Apparatur bestand aus einem 10 m langen beheizten Hauptreaktor (Innendurchmesser: 27 mm) und einem 5,3 m langen beheizten Nachreaktor (Innendurchmesser: 25 mm). Der Kreislaufdurchsatz betrug 25 1/h Flüssigkeit, der Reaktorzulauf wurde auf 4 kg/h eingestellt. Dementsprechend wurden 4 kg/h Hydrieraustrag erhalten.

### Beispiel 1

Das verwendete TMP hatte die Zusammensetzung 22,6 Gew.-% TMP, 1,4 Gew.-% Methanol, 2,1 Gew.-% Trimethylammommiumformiat, 1,1 Gew.-% Methylbutanol, 0,7 Gew.-% Ethylpropandiol, 1,2 Gew.-% Addukten von TMP mit Formaldehyd und Methanol, < 0,1% TMP-Formiat, 1,2 Gew.-% TMP-Dimethylbutanal-Acetale, 2,9 Gew.-% Hochsieder und 66, 2 Gew.-% Wasser. Von dieser Rohlösung wurden 5 kg/h aufgearbeitet. Das Rohgemisch wurde zunächst in der Leichtsiederkolonne bei 400 mbar und 160°C Sumpftemperatur entwässert, wobei der Zulauf in der Mitte der Kolonne erfolgte. Das Rücklaufverhältnis wurde auf 0,3 eingestellt. Aus dem Kolonnensumpf wurden 1,3 kg/h Gemisch bestehend aus 83 Gew.-% TMP, höhersiedenden Verunreinigungen, ca. 1 Gew.-% Wasser und 7,5 Gew.-% TMP-Formiaten entnommen und in die Hochsiederabtrennung überführt. Diese bestand aus einer Kolonne, die bei 20 mbar und einem Rücklaufverhältnis von 0,5 betrieben wurde. Es wurden derart im Kolonnensumpf Hochsieder mit einem Gehalt von 24,4 Gew.-% TMP abgetrennt. Das von Hochsiedern befreite TMP wird am Kopf entnommen und in die Reindestillation verbracht, die aus einer Seitenabzugskolonne besteht, die bei 20 mbar mit temperaturgeregeltem Rücklauf betrieben wird. Die leichter als TMP siedenden Komponenten wie 2-Ethylpropandiol, TMP-Formiat (20,7 Gew.%) werden über Kopf abdestilliert, wobei das Destillat einen Restgehalt von 6,6 Gew.-% TMP aufweist. Zur Vermeidung der Ansammlung von Hochsiedern werden 150 g/h dem Sumpf entnommen und in die Stufe der Hochsiederabtrennung zurückgeführt. Reines TMP mit einem Gehalt von > 99% wird in einer Menge von 1030 g/h am dampfförmigen Seitenabzug oberhalb des Verdampfers entnommen. Die Gesamtausbeute der Aufarbeitung beträgt 90%.

### Beispiel 2

TMP wurde wie in Beispiel 1 beschrieben dargestellt. Es wies die Zusammensetzung 22,1 Gew.-% TMP, 0,4 Gew.-% Methanol, 1,5 Gew.-% Trimethylammoniumformiat, 0,7 Gew.-% Methylbutanol, 0,5 Gew.-% Ethylpropandiol, 1,2 Gew.-% Addukten von TMP mit Formaldehyd und Methanol, < 0,1 Gew.-% TMP-Formiat, 1,4 Gew.-% Acetalen von TMP mit Dimethylbutanal, 2,2 Gew.-% Hochsieder und 69,9 Gew.-% Wasser auf. Der Durchlauf betrug 4 kg/h. Das Rohgemisch wurde zunächst der Leichtsiederkolonne zugeführt, wobei die Entwässerung bei 400 mbar und 180 °C durchgeführt wurde. Der Zulauf erfolgte in der Mitte der Kolonne. Das Rücklaufverhältnis wurde zu 0,3 gewählt. Aus dem Kolonnensumpf wurden 1,1 kg/h entwässertes TMP enthaltend 82,8 Gew.-% TMP, höhersiedende Verunreinigungen, ca. 0,5 Gew.-% Wasser und 6,6 Gew.-% TMP-Formiat abgeführt, mit 40 g/h Dimethylamin vermischt und in einen Rohrreaktor befördert, in dem bei 120°C und einer 1 Std. Verweilzeit gebildetes TMP-Formiat mit Dimethylamin zu TMP und Dimethylformamid umgesetzt wird. Dabei betrug der Umsatz 95%, wodurch der Restgehalt an TMP-Formiat auf < 0,3 Gew.-% gedrückt werden konnte. Der erhaltene Reaktionsaustrag wurde in die Hochsiederabtrennvorrichtung verbracht, wo die Hochsiederabtrennung bei 30 mbar und einem Rücklaufverhältnis von 0 durchgeführt wurde. Dabei wurden im Kolonnensumpf Hochsieder mit einem Restgehalt von 10 Gew.-% TMP abgetrennt. Während der Abtrennung wird diskontinuierlich 85%ige Phosphorsäure in den Kolonnensumpf gegeben, um eine Phosphorsäurekonzentration zwischen 100 ppm und 1000 ppm zu erreichen. Von Hochsiedern befreites TMP wird über Kopf abdestilliert und anschließend in die Reindestillation verbracht, die bei 30 mbar und temperaturgeregeltem Rücklauf in einer Seitenabzugskolonne durchgeführt wurde. In dieser Reindestillation werden leichter als TMP siedende Verbindungen wie 2-Ethylpropandiol oder TMP-Formiat über Kopf abdestilliert, wobei ein Kopfprodukt mit einem Restgehalt von 7 Gew.-% TMP erhalten wird. Dem Sumpf wird in einer Menge von 150 g/h ein Austrag mit einem Gehalt an TMP von > 98 Gew.-% entnommen, der in die Hochsiederabtrennung zurückgeführt wird. Reines TMP mit einem Gehalt von > 99% wird oberhalb des Verdampfers am dampfförmigen Seitenabzug entnommen. Die Gesamtausbeute der Aufarbeitung beträgt 98%, wobei das erhaltene TMP Farbzahlen zwischen 30 und 150 APHA aufwies.

### Beispiel 3

Es wurde wie in Beispiel 2 beschrieben verfahren. Das erhaltene Rein-TMP wurde dann in einer Seitenabzugskolonne einer weiteren Destillation zur Verbesserung der Farbzahl unterworfen. Diese Farbzahldestillation wurde bei 20 mbar und einem Rücklaufverhältnis von 35 durchgeführt. Es wurden 150 g/h Sumpfaustrag mit einem TMP-Gehalt von > 98% und 30 g/h Kopfaustrag mit einem TMP-Gehalt von > 98% in die Reindestillation rückgeführt. Es wurde am dampfförmigen Seitenabzug oberhalb des Verdampfers ein farbloses reines TMP mit einer Farbzahl von 15 bis 50 APHA entnommen.

## Patentansprüche

1. Verfahren zur Reindestillation von aus der Hydrierung von 2, 2-Dimethylolbutanal stammendem Trimethylolpropan, das die folgenden Schritte aufweist:
(a) Umsetzung von n-Butyraldehyd mit Formaldehyd in Gegenwart von katalytischen Mengen eines tertiären Amins und Hydrieren der so erhaltenen Mischung zu einem Trimethylolpropan enthaltenden Gemisch
(b) destillative Abtrennung von Wasser, Methanol, Trialkylamin und/oder Trialkylammoniumformiat bei Drücken von > 200 mbar, vorzugsweise > 400 mbar, Sumpftemperaturen von > 140 °C, vorzugsweise 160 bis 180°C, sowie langen Verweilzeiten so durchgeführt wird, daß zumindest der überwiegende Teil des TMP mit in Schritt (a) gebildetem Trialkylammoniumformiat zu im Sumpf verbleibenden TMP-Formiaten und mit Wasser und Methanol abdestillierendem Trialkylamin reagiert
(c) Erhitzen des in (b) erhaltenen Rückstand im Vakuum auf eine Temperatur, bei der TMP flüchtig ist und höher als TMP siedende Verbindungen zumindest teilweise gespalten werden, unter destillativem Abtrennen von TMP sowie leichter als TMP flüchtigen Verbindungen
(d) Destillation des in (c) erhaltenen Destillats unter Abtrennen der leichter flüchtigen Verbindungen und Gewinnen von Rein-TMP
(e) fakultative Destillation des in (d) erhaltenen Trimethylolpropans zum Gewinnen von TMP mit niedriger APHA-Farbzahl.

2. Verfahren nach Anspruch 1, **dadurch gekennzeichnet, daß** nach dem Schritt (b) ein Schritt (bb) durchgeführt wird, bei dem die gebildeten TMP-Formiate durch Reaktion mit einem niederen Alkohol, vorzugsweise Methanol, oder einem sekundären Amin, vorzugsweise einem Dialkylamin, in TMP und das Formiat des jeweiligen Alkohols beziehungsweise das Formamid des jeweiligen Amins umgewandelt werden.

3. Verfahren nach Anspruch 1 oder 2, **dadurch gekennzeichnet, daß** der Schritt (c) bei Sumpftemperaturen von 210 bis 250°C vorzugsweise 220 bis 235°C, und Drücken von 5 bis 50 mbar, vorzugsweise 10 bis 30 mbar, durchgeführt wird.

4. Verfahren nach einem der Ansprüche 1 bis 3, **dadurch gekennzeichnet, daß** der Schritt (d) bei Sumpftemperaturen von 170 bis 210°C, vorzugsweise 180 bis 200°C und Drücken von 10 bis 40 mbar, vorzugsweise 20 bis 30 mbar, durchgeführt wird.

5. Verfahren nach einem der Ansprüche 1 bis 4, **dadurch gekennzeichnet, daß** der Schritt (e) bei Sumpftemperaturen von 170 bis 210°C, vorzugsweise 180 bis 200°C, und Drücken von 5 bis 30 mbar, vorzugsweise 20 bis 30 mbar, durchgeführt wird.

6. Verfahren nach einem der Ansprüche 1 bis 5, **dadurch gekennzeichnet, daß** die Schritte (b) und (d) derart kombiniert werden, daß das aus dem Schritt (a) stammende Gemisch so aufdestilliert wird, daß sämtliche Verbindungen abdestillieren, die leichter als TMP flüchtig sind, und Rein-TMP in Schritt (c) durch destillatives Abtrennen von den Hochsiedern gewonnen wird.

7. Verfahren nach einem der Ansprüche 1 bis 6, **dadurch gekennzeichnet, daß** die Stufen (c) und (d) zusammengefaßt werden, so daß das Abtrennen der im Sumpf verbleibenden Hochsieder, das Abdestillieren von leichter als TMP flüchtigen Verbindungen und die Gewinnung von Rein-TMP unter Verwenden einer Kolonne mit geeigneter Trennleistung, vorzugsweise einer Kolonne mit Seitenabzug, in einem Schritt durchgeführt wird.

8. Verfahren nach einem der Ansprüche 1 bis 7, **dadurch gekennzeichnet, daß** der Schritt (e) zum Entfernen von farbzahlgebenden Komponenten durchgeführt wird.

## Claims

1. A process for the purification, by distillation, of trimethylolpropane originating from the hydrogenation of 2,2-dimethylolbutanal, said process comprising the following steps:
(a) reaction of n-butyraldehyde with formaldehyde in the presence of catalytic amounts of a tertiary amine, and hydrogenation of the resulting mixture to give a mixture containing trimethylolpropane;
(b) separation of water, methanol, trialkylamine and/or trialkylammonium formate by distillation at pressures of >200 mbar, preferably >400 mbar, at bottom temperatures of >140°C, preferably 160 to 180°C, and with long residence times, in such a way that at least the bulk of the TMP reacts with trialkylammonium formate formed in step (a) to give TMP formates remaining in the bottom of the column and trialkylamine distilled off with water and methanol.
(c) heating of the residue obtained in (b) under reduced pressure to a temperature at which TMP is volatile and compounds boiling above TMP are at least partially cleaved, in order to separate off, by distillation, TMP and compounds more volatile than TMP;
(d) distillation of the distillate obtained in (c) in order to separate off the more volatile compounds and recover pure TMP; and
(e) optional distillation of the trimethylolpropane obtained in (d) in order to recover TMP with a low APHA color index.

2. The process according to claim 1 wherein step (b) is followed by a step (bb) in which the TMP formates produced are converted, by reaction with a lower alcohol, preferably methanol, or a secondary amine, preferably a dialkylamine, to TMP and the formate of the alcohol used or the formamide of the amine used.

3. The process according to claim 1 or 2 wherein step (c) is carried out at bottom temperatures of 210 to 250°C, preferably 220 to 235°C, and pressures of 5 to 50 mbar, preferably 10 to 30 mbar.

4. The process according to any of claims 1 to 3 wherein step (d) is carried out at bottom temperatures of 170 to 210°C, preferably 180 to 200°C, and pressures of 10 to 40 mbar, preferably 20 to 30 mbar.

5. The process according to any of claims 1 to 4 wherein step (e) is carried out at bottom temperatures of 170 to 210°C, preferably 180 to 200°C, and pressures of 5 to 30 mbar, preferably 20 to 30 mbar.

6. The process according to any of claims 1 to 5 wherein steps (b) and (d) are combined so that the mixture originating from step (a) is distilled in such a way that all the compounds more volatile than TMP distill off and pure TMP is recovered in step (c) by distillative separation of the high-boiling components.

7. The process according to any of claims 1 to 6 wherein steps (c) and (d) are combined so that the separation of the high-boiling components remaining in the bottom of the column, the distillation of compounds more volatile than TMP, and the recovery of pure TMP, are carried out in one step using a column of appropriate separation efficiency, preferably a column with a side discharge.

8. The process according to any of claims 1 to 7 wherein step (e) is carried out in order to remove colorizing components.

## Revendications

1. Procédé pour la distillation pure de triméthylolpropane provenant de l'hydruration de 2,2-diméthylolbutanal, qui présente les étapes suivantes :
(a) réaction de n-butyraldéhyde avec du formaldéhyde en présence de mélanges catalytiques d'un amine tertiaire et hydruration du mélange ainsi obtenu pour donner un mélange contenant du triméthylolpropane.
(b) séparation par voie de distillation d'eau, de méthanol, de trialkylamine et/ou de formiate de trialkylammonium à des pressions > 200 mbars, préférentiellement > 400 mbars, des températures de fond > 140°C, préférentiellement de 160 à 180°C, de même que pour des temps de séjour longs, de manière telle qu'au moins la majeure partie du TMP réagisse avec le formiate de trialkylammonium formé au cours de l'étape (a) pour donner des formiates de TMP subsistant dans le fond de la trialkylamine distillée et avec de l'eau et du méthanol.
(c) chauffage du résidu obtenu au cours de l'étape (b) sous vide à une température pour laquelle le TMP est volatil et des composés au point d'ébullition plus élevé que le TMP sont au moins décomposés partiellement sous séparation par voie de distillation du TMP de même que de composés volatils plus légers que le TMP
(d) distillation du distillat obtenu au cours de l'étape (c) avec séparation des composés volatils plus légers et obtention de TMP pur
(e) distillation facultative du triméthylolpropane obtenu au cours de l'étape (d) pour l'obtention de TMP avec un indice de coloration APHA peu élevé.

2. Procédé selon la revendication 1, **caractérisé en ce que** après l'étape (b), une étape (bb) est exécutée au cours de laquelle les formiates de TMP formés, par réaction avec un alcool faible, préférentiellement du méthanol, ou un amine secondaire, préférentiellement un dialkylamine, sont transformés en TMP et le formiate de l'alcool, respectivement, le formamide de l'amide correspondant sont transformés.

3. Procédé selon la revendication 1 ou 2, **caractérisé en ce que** l'étape (c) est exécutée à des températures du fond de 210 à 250°C, préférentiellement de 220 à 235°C et des pressions de 5 à 50 mbars, préférentiellement de 10 à 30 mbars.

4. Procédé selon l'une des revendications 1 à 3, **caractérisé en ce que** l'étape (d) est exécutée à des températures du fond de 170 à 210°C, préférentiellement de 180 à 200°C et des pressions de 10 à 40 mbars, préférentiellement de 20 à 30 mbars.

5. Procédé selon l'une des revendications 1 à 4, **caractérisé en ce que** l'étape (e) est exécutée à des températures du fond de 170 à 210°C, préférentiellement de 180 à 200°C et des pressions de 5 à 30 mbars, préférentiellement de 20 à 30 mbars.

6. Procédé selon l'une des revendications 1 à 5, **caractérisé en ce que** les étapes (b) et (d) sont combinées de manière à ce que le mélange provenant de l'étape (a) est distillé de façon à ce que tous les composés volatils qui sont plus légers que le TMP soient distillés et que du TMP pur soit obtenu au cours de l'étape (c) par séparation par voie de distillation des composés au point d'ébullition élevé.

7. Procédé selon l'une des revendications 1 à 6, **caractérisé en ce que** les étapes (c) et (d) sont rassemblées de manière à ce que la séparation des composés au point d'ébullition élevé restant dans le fond, la distillation de composés volatils plus légers que le TMP et l'obtention de TMP pur avec utilisation d'une colonne à la puissance de séparation appropriée, préférentiellement d'une colonne avec hotte latérale, aient lieu en une seule étape.

8. Procédé selon l'une des revendications 1 à 7, **caractérisé en ce que** l'étape (e) est exécutée pour l'élimination de composants donnant un indice de coloration.
